# EUROPÄISCHE PATENTANMELDUNG

(11) **EP 1 233 363 A2**
(43) Veröffentlichungstag der Anmeldung: **21.08.2002**
(21) Anmeldenummer: 02002774.4
(22) Anmeldetag: 07.02.2002
(51) Int. Cl.: G06F 19/00

(54) **Vernetztes Expertensystem zur automatisierten Befundung und Qualitätskontrolle von medizinischen point-of-care Labormessdaten**

(30) Priorität: 15.02.2001 US 784721
(71) Anmelder: SIEMENS AKTIENGESELLSCHAFT, 80333 München (DE)
(72) Erfinder: Abraham-Fuchs, Klaus, 91058 Erlangen (DE); Hengerer, Arne, Dr., 91054 Erlangen (DE)

(57) **Zusammenfassung**

Vernetztes Expertensystem zur automatisierten Befundung und Qualitätskontrolle von medizinischen Point-of-Care Labormessdaten (POCT), wobei ein POC-Messgerät an einem Ort einer Behandlung über eine Datenleitung oder ein Netzwerk mit einem externen zentralen Expertensystem verbunden ist, das zugleich als virtuelles Labordatenerhebungs- und Diagnosesystem fungiert und das aufgrund implementierter Regeln eine Befundung und Beurteilung der Point-of-Care-Messdaten sowie die Erstellung von Behandlungsvorschlägen für den behandelnden Arzt ermöglicht.

## Beschreibung

Die zentralisierte Labormedizin ist mit einem hohen Logistikaufwand verbunden. Patientenproben müssen zügig und gekühlt in das Zentrallabor geschickt werden, da aufgrund des in der Regel temperatursensiblen Probenmaterials eine Unterbrechung der Kühlkette zu Fehldiagnosen führen kann. Wesentlich kostengünstiger, sicherer und zudem für den niedergelassenen Arzt lukrativer ist es, die Patientenprobe vor Ort, also in der Arztpraxis, zu untersuchen (kostengünstigeres Verschicken von Patientendaten anstatt von Patientenproben). In der POC (Point-of-Care) Diagnostik kann jedoch nur ein begrenztes Spektrum der medizinisch notwendigen Laboruntersuchungen durchgeführt werden. Bei unklaren Testergebnissen können Folgeuntersuchungen in technisch besser ausgestatteten Zentrallaboratorien notwendig werden. Folgeuntersuchungen im Zentrallabor sind aufgrund der damit verbundenen Logistik (durch den Arzt persönlich zu erfolgende initiale Befundung, Beantragung der Folgeuntersuchung, Versendung der Patientenprobe, Kommunikation der Messwerte) mit erheblichem personellen Aufwand und Kosten verbunden. Mit stand-alone-Instrumenten wird das Kosteneinsparungspotential des POC nicht zum Tragen kommen. Einer zusätzlichen Wertschöpfung beim niedergelassenen Arzt wird ein Mehr an Arbeit, unter anderem durch den erheblichen Aufwand bei nicht geplanten, da nicht vorhersehbaren, Folgeuntersuchungen gegenüberstehen. Des Weiteren kann beim behandelnden Arzt nicht in allen Fällen das erforderliche Expertenwissen zur Interpretation der in seiner Praxis generierten Messwerte vorausgesetzt werden. Er kann aufgrund der Wissensexplosion in der molekularen Medizin in manchen Fällen nicht selbständig einen "Laborbefund" erstellen und ist dann auf das Expertenwissen eines Labordiagnostikers angewiesen. Dies gilt insbesondere für genetische Daten, also im Zusammenhang mit der DNA Chip Diagnostik. Diese in der modernen Medizin zunehmende Problemstellung erfordert neue Abläufe bei der Erstellung und Bewertung von labordiagnostischen Daten. So kann z. B. der Labormediziner in der Zukunft seinen niedergelassenen Kollegen sein Expertenwissen mit telemedizinische Kommunikationshilfsmittel zur Verfügung stellen.

Der Erfindung liegt daher die Aufgabe zugrunde, ein System zu schaffen, welches die Vorteile der POC-Diagnostik, also die patientennahe Untersuchung und die fehlende Notwendigkeit, alle Patientenproben zur Untersuchung an ein zentrales Diagnostiklabor übersenden zu müssen, mit den Vorteilen verbindet, die das Expertenwissen in zentralen Diagnostiklabors bietet.

Zur Lösung dieser Aufgabe ist erfindungsgemäß vorgesehen, dass das POC-Messgerät über eine Datenleitung oder ein Netzwerk mit einem externen zentralen Expertensystem verbunden ist, das zugleich als virtuelles Labordatenerhebungs- und Diagnosesystem fungiert und das aufgrund implementierter Regeln eine Befundung und Beurteilung der Point-of-Care-Labormessdaten sowie die Erstellung von Behandlungsvorschlägen für den Arzt ermöglicht.

Durch dieses vernetzte Expertensystem ist ein wesentlich besserer Einsatz vollautomatisierter für die POCT geeigneter Diagnostikinstrumente möglich, wie sie in den nächsten Jahren kommerziell erhältlich sein werden. Jeder behandelnde Arzt kann dann in nennenswertem Umfang mikrobiologische, hämatologische oder klinisch chemische Untersuchungen durchführen, wobei die Befundung durch das externe zentrale Expertensystem von entscheidender Bedeutung ist, da der Arzt selbst mit der Vielzahl der mit den neuen Diagnostikinstrumenten zu gewinnenden Labordaten ohne das notwendige Expertenwissen wenig anfangen könnte.

In Ausgestaltung der Erfindung kann dabei vorgesehen sein, dass das Expertensystem mit einem Zentrallabor verbunden ist, um diesem bei unklaren Diagnoseergebnissen selbsttätig eine Auflistung der notwendigen Folgeuntersuchungen aufzugeben.

Durch diese Vernetzung mit einem Zentrallabor werden beispielsweise dann, wenn die Labordaten unklar sind oder die Befundung aufgrund der gewonnenen Labordaten beim behandelnden Arzt für das Expertensystem nicht erkennbar ist, Folgeuntersuchungen aufgelistet, die zur näheren Abklärung des Krankheitsbildes veranlasst werden sollten. Zusätzlich können noch selbsttätig Patientenproben beim behandelnden Arzt angefordert und ihre Weiterversendung an das Zentrallabor veranlasst werden.

Durch das erfindungsgemäße System ist eine direkte Beratung des behandelnden Arztes durch einen Labormediziner oder Genetiker, beispielsweise via Hotline oder E-Mail, was viel zu zeitaufwendig wäre und daher schwer auf Akzeptanz stoßen würde, entbehrlich. Durch ausgewählte Laborärzte auf dem aktuellen Wissensstand gehaltene Expertensysteme können die Messwerte der POC-Messgeräte beim behandelnden Arzt online auswerten und werden nur in unklaren Fällen ein Zentrallabor mit den dortigen Experten einschalten.

Die erfindungsgemäße telemedizinische POC-Testung erfordert eine Integration der drei Hauptkomponenten Messgerät, Expertensystem und Datenhaltungsmodule. Das POC-Messgerät ist vorzugsweise eine einfache Ausführung der in der Labormedizin gängigen Automaten mit einer an die Arztpraxis angepassten Probendurchsatzkapazität sowie einer konfigurierbaren Schnittstelle. Die Datenübertragung erfolgt vorzugsweise durch Netzwerke, wie beispielsweise das Internet. Die Messwerte werden dann natürlich, ebenso wie sämtliche anderen vertrauliche Patientendaten in einem Datensicherungsmodul durch eine Verschlüsselungssoftware codiert und decodiert. Das Expertensystem basiert vorzugsweise auf Fuzzy-Logic, Bayes'sche Netze, regelbasierten Systemen (z. B. Dr. Gait) oder neuronalen Netzen. In dem Expertensystem werden nicht nur die aktuell bestimmten Labormesswerte berücksichtigt, sondern diese auch mit aktuellem medizinischem Fachwissen "Guidelines", wie sie z. B. aus "Date Warehouses" verfügbar sind, sowie patientenbezogenen Daten aus elektronischen Patientenakten verknüpft. Letzteres sind Datenbanken zur Sammlung aller medizinisch relevanten Patienteninformationen, wobei diese zentral oder dezentral organisiert sein können. Die Datenübertragung geschieht auch hier wie oben beschrieben über Datenleitungen oder Netze, wobei speziell im letzteren Fall stets eine Verschlüsselung erfolgen soll. Eine möglichst zeitlich wie datenbezogen begrenzte Autorisierung und Identifizierung erfolgt vorzugsweise durch den Arzt und den Patienten, beispielsweise durch ein chipkartenbasiertes System.

Die Probengefäße mit den Patientenproben zur Nachuntersuchung im Zentrallabor sollen zur eindeutigen Identifizierung mit einem elektronisch lesbaren Identifizierungsaufdruck, z. B. einem Barcode, versehen sein.

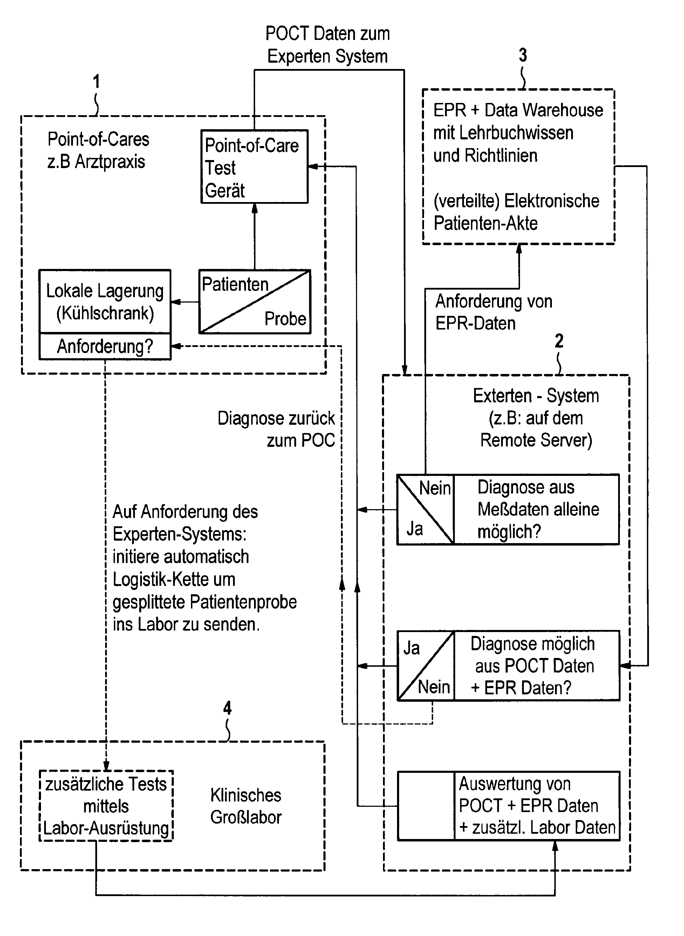

Die Erfindung soll nachstehend anhand eines in der Zeichnung dargestellten Ablaufschemas näher erläutert werden.

### Fall 1:

Der Test wird in einer Point-of-Care-Station 1, beispielsweise in der Arztpraxis durchgeführt. Die Messwerte der Tests werden über eine Datenleitung oder per Internet zu einem zentralen Expertensystem übertragen. Das Messergebnis wird von intelligenten Algorithmen eines Expertensystems als hinreichend für die Erstellung einer eindeutigen Diagnose bewertet. Gegebenenfalls wird das Messergebnis auch unter Berücksichtigung von in elektronischen Patientenakten 3 hinterlegten Daten interpretiert. Der behandelnde Arzt erhält einen Diagnosevorschlag. Eventuell werden aus einem Data Warehouse Therapiekonzepte oder Hintergrundwissen für den Arzt übermittelt. In einer Ausführung der Erfindung kann ein Modul des Data Warehouse bei meldepflichtigen Krankheiten eine Meldung an das zuständige Meldeorgan (z. B. Robert-Koch-Institut in Deutschland) veranlassen. Des Weiteren kann das Data Warehouse auch zur Erhebung epidemiologischer Studien verwendet werden. Diese ermöglicht die frühzeitige Erkennung sich verändernder Resistenzsituationen (Antibiotika- und Antivirale Therapie) oder die Entstehung von Endemien, Epidemien, Pandemien oder Tardivepidemien.

### Fall 2:

Es wird nun angenommen, dass Messwerte der Tests in Analogie zu Fall 1 erhoben, von dem Expertensystem jedoch als nicht ausreichend zur Erstellung einer fundierten Diagnose interpretiert werden. Gründe hierfür können sein, dass Kontrollreaktionen des Tests (Positiv- oder Negativkontrollen) oder elektronische Kontrollen des Geräts als fehlerhaft erkannt werden oder der Verdacht auf eine Erkrankung vorliegt, die in der POC-Station 1 nicht nachgewiesen werden kann. Überdies wird auch der Umstand, dass der POCT keine Anhaltspunkte ergibt, die auf eine bestimmte Erkrankung hinweisen, auch wenn diese aufgrund anderer Patientendaten wahrscheinlich ist, nicht zu einem Diagnosevorschlag führen. In diesen Fällen veranlasst das Expertensystem 2 Anfragen bei Zentrallabors 4 mit ausgewiesener Kompetenz, ob Kapazität für Folgeuntersuchungen besteht. Diese Folgeuntersuchungen können alternativer (gleiche Parameter werden mit anderer Methode bestimmt) oder supplementärer (nicht in der POC-Testung zu bestimmende Parameter werden gemessen) Art sein. Nach positiver Rückmeldung wird in der POC-Station 1, z. B. beim niedergelassenen Arzt, der Versand der, bereits bei der POC-Testung gesplitteten, Patientenprobe veranlasst. Einem Kurierdienst wird z. B. via Internet ein Auftrag erteilt. Das auserwählte Zentrallabor 4 erhält einen detaillierten Anfragebogen. Nach Vermessung der Patientenprobe im Zentrallabor 4 werden die neu generierten Daten in das Expertensystem 2übertragen. Ist anhand der neu vorliegenden Messwerte eine diagnostische Aussage mit signifikanter Wahrscheinlichkeit zu treffen, wird gemäß des unter Fall 1 beschriebenen Modells dem behandelten Arzt ein Diagnosevorschlag unterbreitet. Sind weitere Folgeuntersuchungen zur Erstellung einer hinreichend signifikanten Diagnose notwendig, werden entsprechend den oben skizzierten Schema weitere Folgeuntersuchungen initiiert.

Ein analoges Modell ist für alle Ausprägungen dezentralisierter in vitro Diagnostik möglich, also beispielsweise auch für die Home Care Diagnostik oder in vitro Diagnostik in der Intensiv- und Notfallmedizin.

## Patentansprüche

1. Vernetztes Expertensystem zur automatisierten Befundung und Qualitätskontrolle von medizinischen Point-of-Care Labormessdaten (POCT), **dadurch gekennzeichnet, dass** ein POC-Messgerät an einem Ort einer Behandlung über eine Datenleitung oder ein Netzwerk mit einem externen zentralen Expertensystem verbunden ist, das zugleich als virtuelles Labordatenerhebungs- und Diagnosesystem fungiert und das aufgrund implementierter Regeln eine Befundung und Beurteilung der Point-of-Care-Labormessdaten (POCT) sowie die Erstellung von Behandlungsvorschlägen für den behandelnden Arzt ermöglicht.

2. Expertensystem nach Anspruch 1, **dadurch gekennzeichnet, dass** der Ort der Behandlung in einer Praxis eines zu behandelnden Arztes angeordnet ist.

3. Expertensystem nach Anspruch 1 oder 2, **dadurch gekennzeichnet, dass** das zentrale Expertensystem zur besseren Befundung mit das aktuelle medizinische Wissen enthaltenden Speichersystemen ist.

4. Expertensystem nach einem der Ansprüche 1 bis 3, **dadurch gekennzeichnet, dass** das zentrale Expertensystem zur besseren Befundung mit elektronischen Datenbanken für Patientendaten (EPR) vernetzt ist.

5. Expertensystem nach einem der Ansprüche 1 bis 4, **dadurch gekennzeichnet, dass** das Expertensystem online mit einem Zentrallabor verbunden ist, um diesem bei unklaren Diagnoseergebnissen selbsttätig eine Auflistung von notwendigen Folgeuntersuchungen aufzugeben und Ergebnisse der Folgeuntersuchungen an das Expertensystem zur Neubefundung zurückzumelden.

6. Expertensystem nach Anspruch 5, **dadurch gekennzeichnet, dass** eine Anforderung einer Probe beim Ort der Behandlung und ihre Übersendung zum Zentrallabor automatisch vom Expertensystem veranlasst wird.

7. Expertensystem nach einem der Ansprüche 1 bis 6, **dadurch gekennzeichnet, dass** die verschiedenen Teilsysteme über Internet unter Verwendung von Datenverschlüsselungssystemen miteinander verbunden sind.

8. Expertensystem nach einem der Ansprüche 1 bis 7, **dadurch gekennzeichnet, dass** es mit einem, vorzugsweise chipkartenbasierten Zugangssystem zur Begrenzung des Datenzugriffs auf autorisierte Personen versehen ist.

9. Expertensystem nach einem der Ansprüche 6 bis 8, **dadurch gekennzeichnet, dass** Gefäße mit den Patientenproben mit einem elektronisch lesbaren Identifizierungsaufdruck, z. B. einem Barcode, versehen sind.
